# EUROPEAN PATENT APPLICATION

(11) **EP 3 569 642 A1**
(43) Date of publication of application: **20.11.2019**
(21) Application number: 18739434.1
(22) Date of filing: 11.01.2018
(51) Int. Cl.: C08J 3/12, A61K 8/02, A61K 8/73, A61Q 19/00, A61Q 19/10

(54) **MICROSPHERICAL PARTICLES**

(30) Priority: 13.01.2017 JP 2017004181; 13.01.2017 JP 2017004182
(71) Applicant: Nippon Paper Industries Co., Ltd., Tokyo 114-0002 (JP)
(72) Inventor: KANEKO Yuuma, Tokyo 114-0002 (JP); KOKUFU Yuuki, Tokyo 114-0002 (JP); SAJI Kaoru, Tokyo 114-0002 (JP)
(74) Representative: Hoffmann Eitle
(86) International application number: PCT/JP2018/000411
(87) International publication number: WO 2018/131629

(57) **Abstract**

An object of the present invention is to provide microsphere particles containing powdered cellulose, which have an excellent massage effect, a high cleansing effect, and excellent dispersibility.

A microspherical particle comprising powdered cellulose meets the following condition of (A) or (B):
(A) an average particle diameter of less than 50 µm, and a sphericity of 0.1 or more to 1.0 or less; or
(B) the microspherical particle has an average particle diameter of 50 to 100 µm, and a sphericity of 0.1 or more to less than 0.7.

## Description

### Technical Field

The present invention relates to a microspherical particle including powdered cellulose.

### Background Art

In applications of a cleaning composition such as cleaning cream, and cosmetics, a scrubbing agent has been used to improve cleaning performance and a massage effect. The scrubbing agent is preferred in various countries, especially in the United States.

As such a scrubbing agent, an inorganic pigment such as talc, mica titanium, and kaolin, and a powder of an organic material such as polyethylene are selected and used. In particular, polyethylene beads are used as the scrubbing agent that is excellent in availability of a material, manufacturability, and a massage effect (Patent Literature 1).

However, such a scrubbing agent cannot be removed once discharged into the sewerage due to its very small size and is easily accumulated in the environment because of a lack of biodegradability. As such, there is rising concern over environmental destruction in rivers, ocean, and the like, thus creating a demand for a more environmentally friendly alternative.

As the scrubbing agent having biodegradability, a granulated product using crystalline cellulose (Patent Literature 2) and a method of granulating a powdery material such as biodegradable starch and an anionic binder and coating the granulated product with divalent or higher-valent cations (Patent Literature 3) have been proposed.

### Citation List

### Patent Literature

Patent Literature 1: Patent No. 3032531
Patent Literature 2: Japanese Patent Application Laid-open No. 2003-261436
Patent Literature 3: Japanese Patent Application Laid-open No. 2000-302630

### Summary of Invention

### Technical Problem

However, in Patent Literature 2, a water-soluble binder is used during granulation to prepare a granulated product of the crystalline cellulose. Thus, when the granulated product is added to water-containing cosmetics or the like, the granulated product tends to collapse due to elution of the binder, thereby causing a problem of reduction in the massage effect.

Further, Patent Literature 3 describes that coating of divalent or higher-valent cations after granulation can provide water resistance even if such a water-soluble binder is used. However, since it is in a form of salt, its powdery product is prevented from being uniformly collapsed, thereby causing a problem of reduction in a cleaning effect.

Thus, an object of the present invention is to provide microsphere particles containing powdered cellulose, which have an excellent massage effect, a high cleansing effect, and excellent dispersibility.

### Solution to Problem

That is, the present invention provides the following [1] to [4].
[1] A microspherical particle comprising powdered cellulose, the microspherical particle having the following (A) or (B) :
   (A) an average particle diameter of less than 50 µm, and a sphericity of 0.1 to 1.0; or
   (B) an average particle diameter of 50 to 100 µm, and a sphericity of 0.1 or more to less than 0.7.
[2] The microspherical particle according to the above-mentioned [1], wherein the microspherical particle has an average particle diameter of 5 or more to 70 µm or less, and an average polymerization degree of 50 to 2,000.
[3] A cleaning composition comprising the microspherical particle according to the above-mentioned [1] or [2].
[4] A cosmetic composition comprising the microspherical particle according to the above mentioned [1] or [2].

### Advantageous Effects of Invention

The present invention can provide microsphere particles containing powdered cellulose, which have an excellent massage effect, a high cleansing effect, and excellent dispersibility.

### Description of Embodiments

Hereinafter, the present invention will be described in detail. Note that, unless otherwise specified, the description of "AA to BB" with regard to a numerical range means "AA or more to BB or less" wherein "AA" and "BB" refer to optional numerical values.

### (Microspherical Particle)

The microspherical particle of the present invention contains powdered cellulose as a component.

The microspherical particle of the present invention can be obtained by granulating powdered cellulose described below, and can contain a binder and the like within a range not impairing a desired effect.

Examples of the above-mentioned binders may include an organic binder, and an inorganic system binder, which improve binding force between particles of the powdered cellulose.

However, when such a binder is mixed, depending on conditions such as the type and amount of the binder, it may lead to the contamination of the drainage, or it may affect collapsibility to develop cleaning effect because binding between the particles of the powder cellulose becomes too much strong. However, because the microspherical particle of the present invention can be formed without mixing so-called binder, one preferable embodiment of the present invention includes performing granulation that can give a desired massage feeling without containing a binder.

That is, the microspherical particle of the present invention may be a granulated product without a binder for binding particles of the powdered cellulose to each other. Further, the microspherical particle of the present invention may be a granulated product substantially formed only of the powdered cellulose described above.

As a method of obtaining the microspherical particle of the present invention, a known granulation method capable of producing a spherical particle by granulating the powdered cellulose can be used. As granulation methods, preferred are wet granulation methods such as a tumbling granulation method, a tumbling fluidized granulation method, a centrifugal tumbling granulation method, a fluidized bed granulation method, a stirring tumbling granulation method, a spray drying granulation method, an extrusion granulation method, or a melting granulation method. The tumbling granulation method is more preferable and the centrifugal tumbling granulation method is further preferable to obtain the microspherical particle of the present invention.

In a case of performing such a centrifugal tumbling granulation method, a centrifugal tumbling granulator such as CF-Granulator (manufactured by Freund Corp.) can be used. The rotation number in performing the centrifugal tumbling granulation varies depending on a device in use, but it can be normally range from 100 to 500 rpm.

When the powdered cellulose is charged into the centrifugal tumbling granulator, the powdered cellulose is preferably wetted in advance by adding water or a liquid mainly composed of water, not to be scattered. During the centrifugal tumbling granulation, water or the liquid mainly composed of water is further sprayed on the powdered cellulose. As water or the liquid mainly composed of water, water only or a mixture solution of water and ethanol or the like may be used; however, using only water is preferable to obtain the granulated product having an excellent hardness and specific gravity. It is speculated that when a water ratio in an additive/spray liquid is increased within a balance of not inhibiting a drying process after granulation, the interaction in the cellulose increases and enables the formation of the microspherical particle having an excellent specific gravity and hardness.

Spray conditions (a spray amount, time, and frequency) during such granulation vary depending on the rotation number, an amount of the powdered cellulose as a raw material, and others, and thus cannot be determined generally. However, as an example, the spray conditions can be determined by appropriately adjusting a balance between a slit air rate and the spray liquid after determining the rotation number. For example, the slit air rate can be adjusted within a range of 100 to 400 L/min with respect to 1 kg of the raw material, the spray amount of water can be adjusted within a range of 0.8 to 1.5 kg in total with respect to 1 kg of the raw material, and granulation time can be adjusted within a range of 1 to 4 hours.

Note that, in the present invention, as a method for achieving to fall an average particle diameter of the microspherical particle within a desired range, it is possible to control granulation conditions of the centrifugal tumbling granulator, or to control by subjecting the granulated microspherical particle to a crushing treatment and a classification treatment.

The average particle diameter of the microspherical particle of the present invention is 100 µm or less and the sphericity ranges from 0.1 to 1.0. In such a range, a massage feeling and a cleansing effect can be compatible, and various combinations of forms may be employed depending on conditions such as applications of the microspherical particles.

The upper limit of the average particle diameter of the microspherical particle may be preferably 90 µm or less, 80 µm or less, 70 µm or less, 60 µm or less, 50 µm or less, less than 50 µm, 45 µm or less, 40 µm or less, or 30 µm or less.

The lower limit of the average particle diameter of the microspherical particle may be preferably 5 µm or more, 10 µm or more, 20 µm or more, 30 µm or more, 40 µm or more, 50 µm or more, or 60 µm or more.

The upper limit of the sphericity of the microspherical particle may be 1.0 or less, 0.9 or less, 0.8 or less, 0.7 or less, less than 0.7, 0.65 or less, or 0.5 or less.

The lower limit of the sphericity of the microspherical particle may be 0.1 or more, or 0.2 or more.

The average particle diameter shown in the present invention can be determined, for example, using a laser diffraction/scattering particle size distribution measurement device (for example, Microtrac MT3300EX, manufactured by MicrotracBEL Corp.), by the steps: adding a sample in an amount of 0.2 g to methanol used as a dispersion medium for a measurement; and measuring a particle diameter at a cumulative volume of 50% as the average particle diameter.

The sphericity as used in the present invention can be determined by acquiring image data of the microspherical particle as an observation object using an optical microscope (for example, product name: Digital Microscope VHX-600, manufactured by Keyence Corp.) and then conducting an image analysis with respect to the microspherical particle in the obtained image data by using Image Hyper II (manufactured by Digimo Co., Ltd.). Such a sphericity can be determined from a formula: sphericity = A/B, where A is an area of the microspherical particle obtained by the image analysis, and B is an area of an imaginary perfect sphere with the diameter equal to the maximum major axis diameter of the microspherical particle, which is obtained by calculation. Thus, the microspherical particle has a shape closer to that of a perfect sphere as the sphericity approaches 1. Conversely, the microspherical particle has a more irregular shape as the sphericity draws apart from 1. Note that the sphericity was shown as an average value of 20 microspherical particles observed.

Preferred embodiments of the microspherical particle of the present invention include those of the following (A) or (B):
(A) the microspherical particle has an average particle diameter of less than 50 µm, and a sphericity of 0.1 or more to 1.0 or less;
(B) the microspherical particle has an average particle diameter of 50 to 100 µm, and a sphericity of 0.1 or more to less than 0.7.

In the above embodiment (A), the average particle diameter ranges preferably 5 to 45 µm, more preferably 5 to 40 µm, and further preferably 5 to 30 µm. When the average particle diameter is in these ranges, the dispersibility in the composition is excellent when it is used for a cleaning composition, a cosmetic composition, and the like.

In the above embodiment (A), the sphericity of the microspherical particle of the present invention ranges preferably 0.1 to 0.8, and further preferably 0.1 to 0.5. When the sphericity is in these ranges, both a massage feeling and a cleansing effect are compatible.

In the above embodiment (B), the average particle diameter ranges preferably from 50 to 90 µm, and further preferably from 60 to 90 µm. When the average particle diameter is in these ranges, the dispersibility in the composition is excellent when it is used for a cleaning composition, a cosmetic composition, and the like.

In the above embodiment (B), the sphericity ranges preferably from 0.1 to 0.65 and further preferably from 0.2 to 0.65. When the sphericity is in these ranges, both a massage feeling and a cleansing effect are compatible.

Additionally, the following embodiment (C) is further led as another preferred embodiment of the microspherical particle of the present invention by comprehensively perceiving the embodiments of above (A) and (B) .

(C) the microspherical particle has an average particle diameter of 40 to 90 µm, and a sphericity of 0.1 or more and less than 0.7.

In the above embodiment (C), an average particle diameter ranges from 40 to 90 µm and preferably from 40 to 80 µm and more preferably from 50 to 80 µm. When the average particle diameter is in these ranges, the dispersibility in the composition is excellent when it is used for a cleaning composition, a cosmetic composition, and the like.

In the above embodiment (C), the sphericity is ranges from 0.1 to less than 0.7, preferably from 0.1 to 0.65 and more preferably from 0.2 to 0.5. When the sphericity is in these ranges, both a massage feeling and a cleansing effect are compatible.

The dry hardness in the present invention refers to a load (g/mm²) required for crushing (breaking) one particle of the microspherical particle. Such a dry hardness was determined by measuring a peak value of a crushing strength of one microspherical particle using a particle granule hardness meter (product name: GRANO, manufactured by Okada Seiko Co., Ltd.) and calculating an average value of 20 particles.

The upper limit of the dry hardness of the microspherical particle of the present invention is preferably 210 g/mm² or less, more preferably 200 g/mm² or less, and further preferably 100 g/mm² or less or 50 g/mm² or less. Also, the lower limit of the dry hardness of the microspherical particle of the present invention is preferably 1 g/mm² or more, more preferably 10 g/mm² or more. When the dry hardness is less than 1 g/mm², the microspherical particle easily collapses and thus has a high cleaning effect, but hardly provides the massage feeling. When dry hardness is 210 g/mm² or more, the massaging effect is high, but the microspherical particle less collapses, and thus prospective cleansing effect is hard to be provided.

The microspherical particle of the present invention can be granulated by including an additive, such as a perfume, a disintegration aid, and a granulation accelerating agent, within a range of not inhibiting the desired effect.

### (Powdered Cellulose)

In the present invention, examples of a raw material of the powdered cellulose may include, though not particularly limited to, pulp from a broadleaf tree, pulp from a coniferous tree, pulp from a linter, and non-wood pulp. Preferred is to obtain the powdered cellulose having the small average particle diameter from the viewpoint of convenience in adjusting the granulation of the microspherical particle, and the broadleaf tree pulp having a smaller fiber diameter and fiber width than those of the coniferous tree pulp is preferably used.

Also, in the present invention, examples of a pulping method (a cooking method) may include, though not particularly limited to, a sulfite cooking method, a kraft cooking method, a soda-quinone cooking method, and an organosolv cooking method. Of these, the sulfite cooking method causing a low average polymerization degree is preferable from the viewpoint of environmental aspects.

The powdered cellulose used in the present invention can be obtained by crushing the pulp that has been subjected to an acid hydrolysis treatment with a mineral acid such as hydrochloric acid, sulfuric acid, and nitric acid, or by mechanically crushing the pulp that has not been subjected to an acid hydrolysis treatment.

In a case where the powdered cellulose is obtained by subjecting the pulp raw material described above to the acid hydrolysis treatment and the machine crushing, the powdered cellulose is produced through a raw material pulp slurry preparation step, an acid hydrolysis reaction step, a neutralization-washing-liquid removal step, a drying step, a crushing step, and a classification step.

The pulp raw material can be in a flowable state or in a sheet shape. In a case where flowable pulp from a pulp-bleaching step is used as a raw material, a concentration of the pulp raw material needs to be increased before charging the pulp raw material into a hydrolysis reaction tank. Thus, the pulp raw material is concentrated by a dehydrator such as a screw press and a belt filter and a predetermined amount of the pulp raw material is charged into the reaction tank. In a case where a dry sheet of the pulp is used as a raw material, the pulp is loosened by a crusher such as a roll crusher or the like and then charged into the reaction tank.

Next, a dispersion having a pulp concentration of 3 to 10% by weight (in terms of solid content), which has been adjusted to have an acid concentration of 0.10 to 1.2 N, is treated under conditions of a temperature of 80 to 100°C and a duration of 30 minutes to 3 hours. After the hydrolysis treatment of the pulp, a solid-liquid separation is performed to separate into the hydrolyzed pulp and the waste acid in the dehydration step. The hydrolyzed pulp is neutralized by adding an alkaline agent and washed. Subsequently, the washed product is dried by a dryer, and then mechanically crushed and classified by a crusher into a predetermined size.

Examples of the crusher may include: a cutting type mill such as a mesh mill (manufactured by Horai Co., Ltd.), ATOMS (manufactured by K. K. Yamamoto Hyakuma Seisakusho), a knife mill (manufactured by Pallmann Industries, Inc.), a cutter mill (manufactured by Tokyo Atomizer M.F.G. Co., Ltd.), CS cutter (manufactured by Mitsui Mining Co., Ltd.), a rotary cutter mill (manufactured by Nara Machinery Co., Ltd.), a pulp coarse crusher (manufactured by Zuiko Co., Ltd.), and a shredder (manufactured by Shinko-Pantec Co., Ltd); a hammer type mill such as a jaw crusher (manufactured by Makino Corp.) and a hammer crusher (manufactured by Makino Mfg. Co., Ltd.); an impact mill such as a pulverizer (manufactured by Hosokawa Micron Corp.), Fine Impact Mill (manufactured by Hosokawa Micron Corp.), Super Micron Mill (manufactured by Hosokawa Micron Corp.), Inomizer (manufactured by Hosokawa Micron Corp.), Fine Mill (manufactured by Nippon Pneumatic Mfg. Co., Ltd.), a centrifugal mill (CUM model) (manufactured by Mitsui Mining Co., Ltd.), Exceed Mill (manufactured by Makino Mfg. Co., Ltd.), Ultraplex (manufactured by Makino Mfg. Co., Ltd.), Contraplex (manufactured by Makino Mfg. Co., Ltd.), Kolloplex (manufactured by Makino Mfg. Co., Ltd.), a sample mill (manufactured by Seishin Enterprise Co., Ltd.), a bantam mill (manufactured by Seishin Enterprise Co., Ltd.), an atomizer (manufactured by Seishin Enterprise Co., Ltd.), a tornado mill (manufactured by Nikkiso Co., Ltd.), NEA Mill (manufactured by Dalton Corp.), a fine pulverizer (HT model) (manufactured by Horai Co., Ltd.), Jiyu Mill (manufactured by Nara Machinery Co., Ltd.), New Cosmomizer (manufactured by Nara Machinery Co., Ltd.), a gather mill (manufactured by Nishimura Machine Works Co., Ltd.), Super Powder Mill (manufactured by Nishimura Machine Works Co., Ltd.), Blade Mill (manufactured by Nisshin Engineering Inc.), Super Rotor (manufactured by Nisshin Engineering Inc.), an NPa crusher (manufactured by Sansho Industry Co., Ltd.), a Wiley mill (manufactured by K.K. Miki Seisakusho), a pulp mill (Zuiko Co., Ltd.), Jacobson Mill (manufactured by Shinko-Pantec Co., Ltd.), and a universal mill (manufactured by Tokuju Co., Ltd.); an airflow mill such as a CGS-type jet mill (manufactured by Mitsui Mining Co., Ltd.), Micron Jet (manufactured by Hosokawa Micron Corp.), Counter Jet Mill (manufactured by Hosokawa Micron Corp.), Cross Jet Mill (manufactured by Kurimoto, Ltd.), Supersonic Jet Mill (manufactured by Nippon Pneumatic Mfg. Co., Ltd.), Current Jet (manufactured by Nisshin Engineering Inc.), a jet mill (manufactured by Sansho Industry Co., Ltd.), EBARA Jet Micronizer (manufactured by Ebara Corp.), Ebara Triad Jet (manufactured by Ebara Corp.), Ceren Miller (manufactured by Masuko Sangyo Co., Ltd.), New Microcyclomat (manufactured by Masuno Seisakusho Ltd.), and Kryptron (manufactured by Kawasaki Heavy Industries, Ltd.); and a vertical roller mill such as a vertical roller mill (manufactured by Scenion Inc.), a vertical roller mill (manufactured by Schaeffler Japan Co., Ltd.), a roller mill (manufactured by Kotobuki Engineering & Manufacturing Co., Ltd.), VX Mill (manufactured by Kurimoto, Ltd.), KVM Vertical Mill (manufactured by Earthtechnica Co, Ltd.), and IS Mill (manufactured by IHI Plant Engineering Corp.).

For the purpose of imparting a function to or improving a function of the powdered cellulose of the present invention, the raw material of the powdered cellulose can be mixed with one or two or more other organic and/or inorganic components in an arbitrary ratio, and crushed. Further, a chemical treatment may be applied within a range that does not significantly impair a polymerization degree of natural cellulose used as the raw material.

On the other hand, in a case where the powder is produced only by machine crushing using the pulp as a raw material that has not been subjected to the acid hydrolysis treatment, a vertical roller mill having high fine crushability is preferably used as the crusher. In the present invention, the vertical roller mill refers to a centrifugal vertical crusher belonging to roller mills and performs crushing by grinding a raw material with a discoid turn table and a vertical roller. The most distinctive feature of the vertical roller mill is its excellent fine crushability and, as a reason for this, it can be mentioned that the raw material is crushed by a force to compress the raw material between the roller and the table and a shearing force generated between the roller and the table. Examples of the crusher conventionally used may include a vertical roller mill (manufactured by Scenion Inc.), a vertical roller mill (manufactured by Schaeffler Japan Co., Ltd.), a roller mill (manufactured by Kotobuki Engineering & Manufacturing Co., Ltd.), VX Mill (manufactured by Kurimoto, Ltd.), KVM Vertical Mill (manufactured by Earthtechnica Co, Ltd.), and IS Mill (manufactured by IHI Plant Engineering Corp.).

Note that the powdered cellulose that can be used for preparing the microspherical particle in the present invention is also commercially available.

It is preferable that the powdered cellulose used for the present invention has the average particle diameter of 10 to 50 µm and the average polymerization degree of 50 to 2,000.

An average particle diameter of the powdered cellulose to use for the microspherical particle of the above-mentioned embodiment (A) is preferably 30 µm or less, and more preferably ranges from 5 to 25 µm. When an average particle diameter of the powdered cellulose is less than 5 µm, it becomes difficult to perform the granulation of the microspherical particle due to its fine particle size. On the other hand, when the average particle diameter of the powdered cellulose exceeds 30 µm, it becomes difficult to perform the granulation due to its large particle size.

An average particle diameter of the powdered cellulose to use for the microspherical particle of the above-mentioned embodiment (B) ranges preferably from 5 to 70 µm, and more preferably from 5 to 40 µm. When the average particle diameter of the powdered cellulose is less than 5 µm, it becomes difficult to perform the granulation of the microspherical particle due to its fine particle size. On the other hand, when the average particle diameter of the powdered cellulose exceeds 70 µm, it becomes difficult to perform the granulation due to its large particle size.

An average degree of polymerization of the powdered cellulose of the present invention ranges preferably from 50 to 500, and more preferably from 50 to 200. When the average polymerization degree is greater than the above range, a strength of the powdered cellulose itself becomes high, and thus it is hard to be compressed in granulating and the microspherical particle becomes bulky and the dry hardness becomes insufficient. On the other hand, when the average polymerization degree is lower than the above range, cellulose fibers have less entanglement during the granulation, and thus the microspherical particle becomes inferior in the dry hardness.

The microspherical particle of the present invention is excellent in a massage effect, a cleaning effect, and dispersibility, presumably due to the following reasons. That is, as the average particle diameter becomes larger, a contact area with skin increases, thereby enhancing the massage feeling. However, conventionally, when the average particle diameter of the microspherical particle using cellulose becomes larger, the more binder needs to be formulated for the granulation. Thus, it is considered that this formulation likely causes deformation or the like during the granulation, resulting in impairment of the massage feeling, and reduction in the collapsibility leading to deterioration of the cleaning effect, as well as deterioration of the dispersibility due to the effect of the binder. It is speculated that the microspherical particle of the present invention can simultaneously achieve the massage effect, the cleaning effect, and the dispersibility by maintaining the sphericity and the dry hardness within the predetermined ranges without requiring the binder regardless of the average particle diameter.

### [Cleaning Composition]

The microspherical particle of the present invention can be used by mixing in a cleaning composition together with detergent components having foamability such as body soap, hand soap, and shampoo. Examples of a main detergent component may include those containing a surface active substance such as fatty acid sodium, fatty acid potassium, alpha-sulfo fatty acid ester sodium, sodium linear-alkylbenzene sulfonate, sodium alkyl sulfate ester, sodium alkylether sulfate, sodium alpha-olefin sulfonate, sodium alkyl sulfonate, sucrose fatty acid ester, sorbitan fatty acid ester, polyoxyethylene sorbitan fatty acid ester, fatty acid alkanol amide, polyoxyethylene alkyl ether, polyoxyethylene alkyl phenyl ether, alkylamino fatty acid sodium, alkyl betaine, alkyl amine oxide, alkyl trimethyl ammonium salt, and dialkyl dimethyl ammonium salt. Further, examples of an auxiliary agent may include sodium carbonate, sodium silicate, zeolite, citric acid and salts thereof, EDTA (ethylenediaminetetraacetic acid) and salts thereof, hydroxyethane phosphonic acid, L-aspartic acid diacetic acid (ASDA), L-glutamic acid diacetic acid (GLDA), and sodium sulfate. Furthermore, for example, glycerol, polyethylene glycol, a thickener, a perfume, water, ethanol or the like can be mixed in the cleaning composition, as necessary.

The microspherical particle of the present invention includes the powdered cellulose, which is a chemically stable, as a main component; therefore, the cleaning composition can be formulated without inhibiting the action of the detergent component mentioned above, and the detergent component and the microspherical particle can achieve high cleansing effect.

A type and a mixed amount of the microspherical particles mixed in the cleaning composition may be appropriately set by adjusting according to various conditions such as a type and a dosage form of the cleaning composition, and a specific application. The mixed amount of the microspherical particles in the cleaning composition ranges preferably from 1 to 40% by weight and more preferably from 1 to 30% by weight, which may depending on the dosage form and the like.

### <Cosmetic Composition>

The microspherical particle of the present invention can be mixed in a cosmetic composition. Examples of a cosmetic composition may include those for skin care, body care, face care, and hair care. That is, application sites of the cosmetic composition of the present invention preferably include a face, a lip, a body and a scalp, and the like.

The cosmetic composition may employ various product forms for cosmetics within application for cosmetics without particular limitation. There are various forms for cosmetics to apply to skin, and examples of them may include oil, balm, milky lotion, gel, cream, and a solid stick. Further, an embodiment may be a sheet soaked with, or having a surface attached with, oil, balm, milky lotion, gel, or the like. Such a sheet may be a product of a makeup remover sheet, or the like. Materials such as cotton, a nonwoven fabric cloth, and a paper, which are generally used in the fields for cosmetics and sanitary products, may be used for a sheet substrate.

Various components used for cosmetics may be mixed in a cosmetic composition in addition to the microspherical particle. Examples of components used for the cosmetic composition may include water, alcohol, an oiliness raw material, a surfactant, humectant, a whitening agent, thickening agent, a pH adjustor, ultraviolet absorber, oxidation inhibitor, antiseptics, a sequestering agent (chelating agent), coloring material, perfume, excipient, blood circulation accelerant, a dermatologic preparation agent, a medicine for scalp, other medicinal agents, vitamins, hormones, amino acids, and antihistamine.

A type and a mixed amount of the microspherical particles mixed in the cosmetic composition may be appropriately set by adjusting according to various conditions such as a type and a dosage form of the cosmetic composition, and a specific application. The mixed amount of the microspherical particle in a cosmetic composition ranges preferably from 1 to 50% by weight and more preferably from 1 to 40% by weight, which may depending on the dosage form and the like.

### Examples

The present invention will be described in detail below by way of examples; however, the present invention is not limited by the following examples.

### <Example 1-1 (Microspherical Particle 1)>

Powdered cellulose W-06MG (manufactured by Nippon Paper Industries Ltd., mean particle size 6 µm, average degree of polymerization 150, apparent specific gravity 0.34 g/ml) in an amount of 500 g was charged into a mixer, and water was adequately added and they were mixed by stirring. This wetted powder was charged into a centrifugal tumbling granulator CF-360N (manufactured by Freund Corp.) and granulation was performed with spraying water appropriately during 100 minutes. The generated particles were fluidized and dried, thus obtaining microspherical particles having an average particle diameter of 50 µm, a sphericity of 0.68, a dry hardness of less than 20 g/mm², and an apparent specific gravity of 0.38 g/ml.

### <Example 1-2 (Microspherical Particle 2)>

Microspherical particles having an average particle diameter of 24 µm, a sphericity of 0.65, a dry hardness of less than 20 g/mm², and an apparent specific gravity of 0.80 g/ml were obtained in the same manner as that in Example 1-1 except that the time for granulation was increased.

### <Example 1-3 (Microspherical Particle 3)>

Microspherical particles having an average particle diameter of 32 µm, a sphericity of 0.60, a dry hardness of less than 20 g/mm², and an apparent specific gravity of 0.63 g/ml were obtained in the same manner as that in Example 1-1 except that the amount of spraying water was increased.

### <Comparative Example 1>

Polyethylene beads (product name: Microscrub 35PC, manufactured by Prospector Corp.) having an average particle diameter of 350 µm and a sphericity of 0.38 was used instead of the microspherical particles containing the powdered cellulose.

### <Example 2-1 (Microspherical Particle 4)>

Powdered cellulose W-06MG (manufactured by Nippon Paper Industries Ltd., mean particle size 6 µm, average degree of polymerization 150, apparent specific gravity 0.34 g/ml) in an amount of 500 g was charged into a mixer, and water was adequately added and they were mixed by stirring. This wetted powder was charged into a centrifugal tumbling granulator CF-360N (manufactured by Freund Corp.) and granulation was performed with spraying water appropriately during 100 minutes. The generated particles were fluidized and dried, thus obtaining microspherical particles having an average particle diameter of 62 µm, a sphericity of 0.69, a dry hardness of less than 20 g/mm², and an apparent specific gravity of 0.51 g/ml.

### <Example 2-2 (Microspherical Particle 5)>

Powdered cellulose W-400M (manufactured by Nippon Paper Industries Ltd., mean particle size 24 |um, average degree of polymerization 140, apparent specific gravity 0.48 g/ml) in an amount of 500 g was charged into a mixer, and water was adequately added and they were mixed by stirring. This wetted powder was charged into a centrifugal tumbling granulator CF-360N (manufactured by Freund Corp.) and granulation was performed with spraying water appropriately during 100 minutes. The generated particles were fluidized and dried, thus obtaining microspherical particles having an average particle diameter of 88 µm, a sphericity of 0.69, a dry hardness of less than 20 g/mm², and an apparent specific gravity of 0.74 g/ml.

### <Example 2-3 (Microspherical Particle 6)>

Microspherical particles having an average particle diameter of 58 µm, a sphericity of 0.70, a dry hardness of less than 20 g/mm², and an apparent specific gravity of 0.66 g/ml were obtained in the same manner as that in Example 2-1 except that the amount of spraying water was decreased.

### <Comparative Example 2>

As another cellulose type of a microspherical particle, cellulose beads (VIVAPURCS100S, manufactured by J. Rettenmaier & Sohne), having an average particle diameter 170 µm, dry hardness 84 g/mm², were used.

### <Evaluation of Microspherical Particle>

### <Average Particle Diameter>

A laser diffraction/scattering particle size distribution measurement device (Microtrac MT3300EX, manufactured by MicrotracBEL Corp.) was used. A measurement was performed with a sample in an amount of 0.2 g, which was added to ethanol used as a dispersion medium in the measurement, thus determining a particle diameter at a cumulative volume of 50% (the average particle diameter).

### <Measurement of Sphericity>

Image data of the microspherical particle as an observation object was acquired using an optical microscope (product name: Digital Microscope VHX-600, manufactured by Keyence Corp.) and image analysis was performed using Image Hyper II (manufactured by Digimo Co., Ltd.). The sphericity was determined from the formula: sphericity = A/B, where A was an area of the microspherical particle determined by the image analysis, and B was an area of an imaginary perfect sphere which is determined by calculation assuming that the diameter of which is the maximum major axis diameter of the microspherical particle.

### <Measurement of Dry Hardness>

A dry hardness (g/mm²) was determined by measuring a peak value of a crushing strength of one microspherical particle using a particle granule hardness meter (product name: GRANO, manufactured by Okada Seiko Co., Ltd.) and calculating an average value of 20 particles.

### <Cleaning Composition>

### <Cleaning Composition, Evaluation of Massage (Body)>

To 95 g of a commercially available body cleanser (product name: Dove body wash G, manufactured by Unilever Japan K.K.), 5 g of the microspherical particles of Examples 1-1 to 1-3 and 2-1 to 2-3, the polyethylene beads of Comparative Example 1, or a cellulose type microspherical particle (product name: VIVAPURCS100S, manufactured by J. Rettenmaier & Sohne) of Comparative Example 2 were each added and mixed by stirring, thus preparing each mixture liquid. After the mixture liquids thus obtained were left to stand for 5 hours, 5 g of each mixture liquid was applied to the cheeks of five subjects and then the applied part was rubbed 20 times by the palm. Then, feeling of rubbing was evaluated according to the following indexes. The evaluation was indicated with the average value of the five subjects.
A: Tactile sensation with massage feeling was given.
B: Tactile sensation with weak massage feeling was given.
C: No tactile sensation with no massage feeling was given.

Results of each body cleaning composition including Examples 1-1 to 1-3 or the polyethylene beads of Comparative Example 1 are shown in Table 1. Further, results of each cleaning composition including Examples 2-1 to 2-3 or a cellulose type microspherical particle of Comparative Example 2 are shown in Table 2.

Note that, in Tables 1-3 shown below, the symbol of "-" indicates non-measurement or measurement inability.

### <Cleaning Composition, Evaluation of Massage (Scalp)>

To 95 g of a commercially available body cleanser (product name: Merit, manufactured by Kao Corp.), 5 g of the microspherical particles of Examples 1-1 to 1-3 and 2-1 to 2-3, the polyethylene beads of Comparative Example 1, or cellulose type microspherical particles (product name: VIVAPURCS100S, manufactured by J. Rettenmaier & Sohne) of Comparative Example 2 were each added and mixed by stirring, thus preparing each mixture liquid. After the mixture liquids thus obtained were left to stand for 5 hours, 0.5 g of each mixture liquid was applied to a scalp of five subjects and then the applied part was rubbed 10 times by a finger. Then, feeling of rubbing was evaluated according to the following indexes. The evaluation was indicated with the average value of the five subjects.
A: Tactile sensation with massage feeling was given.
B: Tactile sensation with weak massage feeling was given.
C: No tactile sensation with no massage feeling was given.

Results of each body cleaning composition including Examples 1-1 to 1-3 or the polyethylene beads of Comparative Example 1 are shown in Table 1. Further, results of each cleaning composition including Examples 2-1 to 2-3 or cellulose type microspherical particles of Comparative Example 2 are shown in Table 2.

### <Cleaning Composition, Evaluation of Massage (inside mouth)>

To 95 g of a commercially available toothpaste (product name: Guard Hello Standing Tube, manufactured by Kao Corp.), 5 g of the microspherical particles of Examples 1-1 to 1-3 and 2-1 to 2-3, the polyethylene beads of Comparative Example 1, or cellulose type microspherical particles (product name: VIVAPURCS100S, manufactured by J. Rettenmaier & Sohne) of Comparative Example 2 were each added and mixed by stirring, thus preparing each mixture liquid. After the mixture liquids thus obtained were left to stand for 5 hours, five subjects each took 1 g of each mixture liquid by a finger and applied to inside of the mouth and the gums, and then the applied part was rubbed 10 times. Then, feeling of rubbing was evaluated according to the following indexes. The evaluation was indicated with the average value of the five subjects.
A: Tactile sensation with massage feeling was given.
B: Tactile sensation with weak massage feeling was given.
C: No tactile sensation with no massage feeling was given.

Results of each body cleaning composition including Examples 1-1 to 1-3 or the polyethylene beads of Comparative Example 1 are shown in Table 1. Further, results of each cleaning composition including Examples 2-1 to 2-3 or a cellulose type microspherical particle of Comparative Example 2 are shown in Table 2.

### <Cleaning Composition, Evaluation of Cleaning Performance (body)>

To 95 g of a commercially available body soap (product name: Biore uRf, manufactured by Kao Corp.), 5 g of the microspherical particles of Examples 1-1 to 1-3 and 2-1 to 2-3 were each added, thus preparing each cleaning liquid. An area measuring 2 x 2 cm on the left palm of each panelist was uniformly painted with a blue oily marking pen (Hi-Mackee Care, manufactured by Zebra Co., Ltd.). Subsequently, 5 g of the above cleaning liquids were each applied to the painted part to clean by rubbing 100 times with both palms. After washed with water and dried, the palm was observed with 20x magnification using a microscope (VH-7000, manufactured by Keyence Corp.) to evaluate a removal degree (the cleaning performance) of the blue marking. Results are shown in Table 1.
A+: Cleaning performance was very good. Most of blue color was removed.
A: The blue color was so removed that cleaning performance was realized.
B: Cleaning performance was observed, but blue color faintly remained.
C: Cleaning performance was observed and blue color remained.

### <Cleaning Composition, Evaluation of Dispersibility>

An amount of 100 g each of the cleaning liquids, which were prepared as described above, was placed in a screw-top glass bottle (250 ml). After the lid was put, the bottle was shaken up and down ten times. After the bottle was left to stand for 1 hours, the lid was opened and the liquid was stirred again with a glass stir bar, and then dispersibility was evaluated according to the following manners.
A: There was no resistance at the time of stirring at the bottom part of the screw-top glass bottle, and the dispersibility of the microspherical particles was excellent.
B: A little resistance was felt at the time of stirring at the bottom part of the screw-top glass bottle. Some sediment of the microspherical particles was felt; however, it was solved by the re-stirring.
C: Resistance was felt at the bottom part of the screw-top glass bottle. It was not solved by the re-stirring.

### [Table 1]

**Table 1: Evaluation of Microspherical Particle 1-3 (Cleaning Composition)**

| Type of Microspherical Particle | Material of Microspherical Particle | Microspherical Particle | | | Body | | | Scalp | Inside Mouth |
|---|---|---|---|---|---|---|---|---|---|
| | | Average Particle Diameter (µm) | Sphericity | Dry Hardness (g/mm²) | Massage Effect | Evaluation of Cleaning | Evaluation of Dispersibility | Massage Effect | Massage Effect |
| Microspherical Particle 1 (Example 1-1) | W-06MG | 50 | 0.68 | less than 20 | A | A+ | A | A | A |
| Microspherical Particle 2 (Example 1-2) | W-06MG | 24 | 0.65 | less than 20 | B | A+ | A | B | A |
| Microspherical Particle 3 (Example 1-3) | W-06MG | 32 | 0.60 | less than 20 | A | A | A | B | A |
| Polyethylene Beads (Comparative Example 1) | | 350 | 0.38 | - | A | - | - | A | A |

### [Table 2]

**Table 2: Evaluation of Microspherical Particle 4-6 (Cleaning Composition)**

| Type of Microspherical Particle | Material of Microspherical Particle | Microspherical Particle | | | Body | | | Scalp | Inside Mouth |
|---|---|---|---|---|---|---|---|---|---|
| | | Average Particle Diameter (µm) | Sphericity | Dry Hardness (g/mm²) | Massage Effect | Evaluation of Cleaning | Evaluation of Dispersibility | Massage Effect | Massage Effect |
| Microspherical Particle 4 (Example 2-1) | W-06MG | 62 | 0.69 | less than 20 | A | A+ | A | A | A |
| Microspherical Particle 5 (Example 2-2) | W-400M | 88 | 0.69 | less than 20 | A | A+ | B | A | A |
| Microspherical Particle 6 (Example 2-3) | W-06MG | 58 | 0.70 | less than 20 | A | A+ | A | B | A |
| VIVAPUR CS100S (Comparative Example 2) | Cellulose | 170 | - | 84 | A | B-C | B | B | B-C |
| Polyethylene Beads (Comparative Example 1) | | 350 | 0.38 | - | A | A | A | A | A |

### <Cosmetic Composition>

### <Cream>

To 10 g of commercial moisturizing cosmetic (product name: NIVEA Cream c, manufactured by Nivea Kao Corp.), 2 g of the microspherical particles of Example 2-2 (Microspherical Particle 5), Example 2-6 (Microspherical Particle 6), Example 1-3 (Microspherical Particle 3), or Example 1-2 (Microspherical Particle 2), as mentioned above, were each added and stirred well to prepare a mixture liquid, thereby obtaining each cream agent.

### <Solid Stick>

To a surface for use of a commercial moisturizing cosmetics in the form of a solid stick (product name: Menturm medical use stick, manufactured by Omi Brothers Corp.), 2 g of the microspherical particles of Examples 2-2 (Microspherical Particle 5), Example 2-6 (Microspherical Particle 6), Example 1-3 (Microspherical Particle 3) or Example 1-2 (Microspherical Particle 2), as mentioned above, were each uniformly attached, thereby obtaining each solid stick agent.

### <Milky Lotion>

To 10 g of a commercial moisturizing cosmetic (product name: Grandane Luxage Lift Moisture Emulsion, manufactured by Kose Corp.), 2 g of the microspherical particles of Example 2-2 (Microspherical Particle 5), Example 2-6 (Microspherical Particle 6), Example 1-3 (Microspherical Particle 3) or Example 1-2 (Microspherical Particle 2), mentioned above, were each added and stirred well to prepare a mixture liquid, thereby obtaining each milky lotion agent.

### <Oil>

To 10 g of a commercial moisturizing cosmetic (product name: Johnson Baby Oil, manufactured by Johnson & Johnson Corp.), 3 g of the microspherical particles of Examples 2-2 (Microspherical Particle 5), Example 2-6 (Microspherical Particle 6), Example 1-3 (Microspherical Particle 3) or Example 1-2 (Microspherical Particle 2), mentioned above, were each added and stirred well to prepare a mixture liquid, thereby obtaining each oil agent.

### <gel>

To 10 g of a commercial moisturizing cosmetic (product name: Chifure Wet and Soft Gel, manufactured by Chifure Corp.), 2 g of the microspherical particles of Example 2-2 (Microspherical Particle 5), Example 2-6 (Microspherical Particle 6), Example 1-3 (Microspherical Particle 3), or Example 1-2 (Microspherical Particle 2), mentioned above, were each added and stirred well to prepare a mixture liquid, thereby obtaining each gel agent.

### <Cosmetic Composition, Evaluation of Massage (Body) >

Each cosmetic composition of the cream, the solid stick, the milky lotion, the oil, or the gel was applied at an appropriate amount on the brachium and elbow of five subjects by lightly stroking. Then, feeling of application was evaluated according the following indexes. The evaluation was indicated with the average value of the five subjects. Evaluation results are shown in Table 3.
A: Tactile sensation with massage feeling was given.
B: Tactile sensation with weak massage feeling was given.
C: No tactile sensation with no massage feeling was given.

Note that when two evaluation indexes are tied with the symbol of "-", it means that its evaluation is at the middle of these two indexes. For example, when an evaluation is between A and B, it is described as "A-B."

### <Cosmetic composition, Evaluation of Wiping Off Feeling>

Furthermore, a coated surface was wiped off once with a tissue (product name: Scottie, manufactured by NIPPON PAPER CRECIA Corp.). Then, feeling of wiping off was evaluated according the following indexes. The evaluation was indicated with the average value of five subjects. Evaluation results are shown in Table 3.
A: There was no or less feeling of friction at the time of wiping off.
B: There was a little friction feeling at the time of the wiping off.
C: There is strong friction feeling at the time of the wiping off, which causes pain.

### <Cosmetic Composition, Evaluations of Massage (Face) and Wiping Off Feeling>

The massage feeling and wiping off feeling were evaluated in the same manner as described above, by using each cosmetic composition of the cream, the solid stick, the milky lotion, the oil, or the gel which were prepared as described above, except that it was evaluated at cheek and T-zone, which is the part from forehead to nose, of five subjects. Those were shown in Table 3 in the average value of the five subjects in the same manner as above. Evaluation results are shown in Table 3.

### [Table 3]

**Table 3: Evaluation of Cosmetic Composition**

| Microspherical Particle | | | Form of Cosmetices | Body | | Face | |
|---|---|---|---|---|---|---|---|
| Type of Microspherical Particle | Average Particle Diameter (µm) | Sphericity | | Massage Effect | Wiping off Feeling | Massage Effect | Wiping Off Feeling |
| Microspherical Particle 5 | 88 | 0.69 | Cream | A | A-B | A | A-B |
| Microspherical Particle 6 | 58 | 0.70 | | A-B | A-B | A-B | A-B |
| Microspherical Particle 3 | 32 | 0.60 | | A-B | A-B | A-B | A-B |
| Microspherical Particle 2 | 24 | 0.65 | | A-B | A-B | A-B | A-B |
| Microspherical Particle 5 | 88 | 0.69 | Solid Stick | A | A-B | A | A-B |
| Microspherical Particle 6 | 58 | 0.70 | | A-B | A-B | A-B | A-B |
| Microspherical Particle 3 | 32 | 0.60 | | A-B | A-B | A-B | A-B |
| Microspherical Particle 2 | 24 | 0.65 | | A-B | A-B | A-B | A-B |
| Microspherical Particle 5 | 88 | 0.69 | Milky Lotion | A-B | A-B | A-B | A-B |
| Microspherical Particle 6 | 58 | 0.70 | | A-B | B | A-B | B |
| Microspherical Particle 3 | 32 | 0.60 | | A-B | B | A-B | B |
| Microspherical Particle 2 | 24 | 0.65 | | A-B | B | A-B | B |
| Microspherical Particle 5 | 88 | 0.69 | Oil | A | A-B | A | A-B |
| Microspherical Particle 6 | 58 | 0.70 | | A-B | B | A-B | B |
| Microspherical Particle 3 | 32 | 0.60 | | A-B | B | A-B | B |
| Microspherical Particle 2 | 24 | 0.65 | | A-B | B | A-B | B |
| Microspherical Particle 5 | 88 | 0.69 | Gel | A | A-B | A | A-B |
| Microspherical Particle 6 | 58 | 0.70 | | A-B | B | A-B | B |
| Microspherical Particle 3 | 32 | 0.60 | | A-B | B | A-B | B |
| Microspherical Particle 2 | 24 | 0.65 | | A-B | B | A-B | B |

## Claims

1. A microspherical particle comprising powdered cellulose, the microspherical particle having the following (A) or (B) :
(A) an average particle diameter of less than 50 µm, and a sphericity of 0.1 to 1.0; or
(B) an average particle diameter of 50 to 100 µm, and a sphericity of 0.1 or more to less than 0.7.

2. The microspherical particle according to claim 1, wherein the microspherical particle has an average particle diameter of 5 or more to 70 µm or less, and an average polymerization degree of 50 to 2,000.

3. A cleaning composition comprising the microspherical particle according to claim 1 or 2.

4. A cosmetic composition comprising the microspherical particle according to claim 1 or 2.
